# EUROPEAN PATENT SPECIFICATION

(11) **EP 0 834 498 B1**
(45) Date of publication and mention of the grant of the patent: **31.05.2000**
(21) Application number: 97307688.8
(22) Date of filing: 30.09.1997
(51) Int. Cl.: C07C 209/08

(54) **Process for the preparation of aminoalkynes**
Verfahren zur Herstellung von Aminoalkynen
Procédé de préparation d'aminoalkynes

(30) Priority: 01.10.1996 US 26864
(43) Date of publication of application: 08.04.1998
(73) Proprietor: ROHM AND HAAS COMPANY, Philadelphia, Pennsylvania 19106-2399 (US)
(72) Inventor: Stephens, Randall Wayne, Perkasie, Pennsylvania 18944 (US); Roemmele, Renee Caroline, Maple Glen, Pennsylvania 19002 (US)
(74) Representative: Buckley, Guy Julian

(56) References cited:
- EP-A- 0 099 302
- FR-A- 2 048 865
- US-A- 5 254 584
- C. F. HENNION ET AL: "The preparation of some acetylenic primary amines" JOURNAL OF THE AMERICAN CHEMICAL SOCIETY, vol. 75, 1953, DC US, pages 1653-1654, XP002051447

## Description

This invention relates to an improved process for the preparation of amines which are useful in the subsequent formation of biologically active materials.

The process of this invention comprises a two step sequence wherein an alcohol is converted to an organic chloride through reaction with a chlorinating agent in a first step followed by amination of the organic chloride to the corresponding amine in a second step. In the second step, a surfactant is employed in order to facilitate the reaction. The presence of the surfactant is especially valuable when the organic chloride which is formed in the first step is water insoluble but the amination agent is soluble in water. While not wanting to be bound by theory, it is believed that the surfactant increases the surface area of the organic chloride in the aqueous medium. This results in a larger contact area for reaction with the amination agent and consequently improves the mass transfer ability; thus, the time of reaction is reduced. Additionally, the selectivity and the yield of desired amine material is increased because of reduced formation of undesirable by-products; this results in an economically viable process and the ability to offer the subsequent pesticidal product to the marketplace in a more economical fashion.

The process of this invention, employing a surfactant, is most useful for the preparation of aminoalkynes from the corresponding chloroalkyne and its precursor alkynyl alcohol wherein the chloroalkyne is water insoluble and the amination agent, such as ammonia or methylamine, is water soluble. Processes for the amination of chloroalkynes have been described by both Michelotti et al. in US 5,254,584 and Hennion et al. in *J. Am. Chem. Soc.,* **75**, 1653 (1953); however, the use of a surfactant in such processes was not disclosed or suggested. Another method of preparing an aminoalkyne by reaction of the chloroalkyne with sodium amide in liquid ammonia also has been disclosed in *J. Org. Chem.,* **45**, 4616 (1980); however, such processes are only viable on a laboratory scale and are not suitable as a large scale commercial process.

The process of the present invention comprises the steps of
a. reacting an alkynyl alcohol with HCl to form a chloroalkyne,
b. reacting said chloroalkyne with a water soluble amination agent in the presence of a surfactant to form an aminoalkyne and, optionally,
c. purifying said aminoalkyne by distillation.

More specifically, the process of this invention comprises the steps of
a. reacting an alkynyl alcohol of the formula 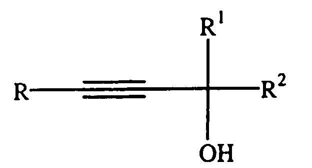 with an aqueous, saturated HCl solution to form a chloroalkyne of the formula 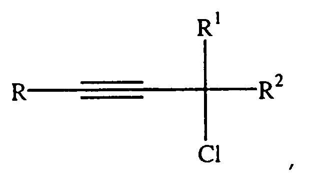
b. reacting said chloroalkyne with a water soluble amination agent of the formula NR³R⁴ in the presence of a non-ionic, cationic or amphoteric surfactant to form an aminoalkyne of the formula 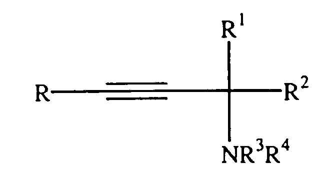 and, optionally,
c. purifying said aminoalkyne by distillation;
wherein
R is a hydrogen atom, alkyl, cycloalkyl, cycloalkylalkyl or aralkyl;
R¹ and R² are each independently alkyl, cycloalkyl, cycloalkylalkyl, aralkyl or, together with the carbon atom to which they are attached, form cycloalkyl; and
R³ and R⁴ are each independently a hydrogen atom or a lower alkyl.

In this invention, alkyl is straight or branched chain (C₁-C₈)alkyl and includes, for example, methyl, ethyl, *n*-propyl, isopropyl, *n*-butyl, isobutyl, *sec*-butyl, *tert*-butyl, *n*-amyl, isoamyl, *n*-hexyl and *n*-octyl. Lower alkyl is straight or branched chain (C₁-C₄)alkyl. Cycloalkyl includes, for example, cyclopentyl and cyclohexyl. Cycloalkylalkyl includes, for example, cyclopentylmethyl, cyclohexylethyl, 3-cyclopentylpropyl, 4-cyclohexylbutyl and the like. For aralkyl, the aryl part of the moiety is defined as phenyl or phenyl substituted with one or two substituents independently selected from halo and alkyl; the alkyl part of the moiety is defined as straight chain (C₁-C₄)alkyl. Examples of aralkyl include benzyl, phenethyl, 4-chlorobenzyl, 4-methylbenzyl and 2-chlorophenethyl.

In a preferred embodiment of this invention, R is a hydrogen atom or lower alkyl, R¹ and R² are independently lower alkyl or, together with the carbon atom to which they are attached, form cyclopentyl or cyclohexyl, R³ and R⁴ are independently a hydrogen atom or lower alkyl, and the surfactant is non-ionic. In a more preferred embodiment of this invention, R is a hydrogen atom, R¹ and R² are independently methyl or ethyl, R³ and R⁴ are both hydrogen atoms, and the surfactant is an alkylphenoxy polyethoxy ethanol. In an even more preferred embodiment of this invention, R¹ is methyl and R² is methyl or ethyl.

The reaction sequence for both steps 1 and 2 is most conveniently carried out at ambient pressure and a temperature of from 10°C to -10°C. However, if desired, the sequence can be run under pressure greater than atmospheric and at higher temperatures. The stoichiometry is relatively unimportant, but it is generally most convenient to employ a stoichiometric excess of HCl in the first step and a stoichiometric excess of amination agent in the second step. A chlorination catalyst such as copper (I) chloride may be utilized in the first step. A hydrogen chloride sink, for example, a strong base such as sodium or potassium hydroxide, is generally used in the second step to reduce consumption of the amination reagent; however, an excess of amination reagent can also be employed as the HCl sink if desired. The reaction times for both steps can vary and are usually dependent upon the cooling capacity and the mixing characteristics of the reaction vessel; conversion of starting material to the desired intermediate or product is conveniently followed using gas liquid chromatography (GLC) or high performance liquid chromatography (HPLC). The amount of surfactant utilized in step 2 can be varied but is generally within the range of 0.01 - 10% by weight based on the amount of organic chloride present. Preferably, the amount of surfactant will be within the range of 0.1 - 1.0% by weight based on the amount of organic chloride present.

The following examples are meant to further illustrate the present invention.

### Example 1: Formation of 3-Chloro-3-methyl-1-pentyne

To a reactor, consisting of a 1-L resin kettle equipped with a thermometer, gas dispersion tube, overhead stirring motor with a retreat agitator blade, Lauda type circulating bath, caustic scrubber and a pressure equalizing addition funnel with attachment for a slow nitrogen sweep, was added 300 mL (3.6 mol) of concentrated hydrochloric acid along with 1.58 g (16 mmol) of copper (I) chloride. The cooling bath was set to 0°C, and hydrogen chloride gas was introduced. The dissolution of HCl was exothermic; as the solution approached saturation, the bath temperature was lowered further until a kettle temperature of - -5°C was reached. The 3-methyl-1-pentyn-3-ol (250 g, 2.5 mol) was charged to the addition funnel. A slow nitrogen sweep was started to keep the HCl vapor from reaching the alcohol through the side arm. The alcohol was added dropwise at a rate such that the reaction temperature remained at 0°C or less for 2-3 hours. Addition of hydrogen chloride gas was continued during the feed to maintain saturation. At the end of the feed, the hydrogen chloride addition was stopped and the dispersion tube elevated above the level of the liquid. The reaction mixture was allowed to stir for 30 minutes at 0°C, then the agitation was stopped and the layers allowed to separate. The lower aqueous layer was drawn off and the organic phase was washed with water and then with a mixture of saturated brine and sodium bicarbonate solution. The organic phase was stored in a refrigerator until used for the next step. The procedure afforded about 280-290 g of a yellow to brown liquid whose estimated purity by GLC was found to be between 90-96%.

### Example 2: Formation of 3-Amino-3-methyl-1-pentyne

To a reactor, consisting of a 1-liter resin kettle equipped with an overhead stir motor, Lauda type refrigerated bath, gas dispersion tube, two pressure equalizing addition funnels, thermometer, nitrogen sweep, and a gas inlet and exit equipped with the appropriate traps, was added 350 mL (2.6 mol) of concentrated ammonium hydroxide solution and 1.00g of Triton® X-100 (Footnote 1). The cooling bath was set to approximately 0°C and ammonia gas was introduced. The dissolution of ammonia was quite exothermic. As the solution approached saturation, the bath temperature was lowered further until a kettle temperature of ∼ -5°C was reached. One of the addtion funnels was charged with 250 g (2.0 mol) of 3-chloro-3-methyl-1-pentyne and the other was charged with 172 g (2.2 mol) of 50% sodium hydroxide. A slow nitrogen sweep was placed on the chloride containing addition funnel to prevent ammonia vapors from entering the funnel through the side arm. The chloride and caustic were simultaneously added dropwise at a rate such that the reaction temperature remained at 0°C or less for 3-4 hours. Addition of ammonia gas was continued during the feed to maintain saturation. At the end of the feed, ammonia addition was stopped and the gas dispersion tube elevated above the level of the liquid. The reaction mixture was allowed to stir at 0°C until the level of unreacted chloride was found to be less than 1% by GLC. Agitation was stopped and the layers allowed to separate. The lower aqueous layer was dawn off and discarded and the upper organic layer was transferred to a distillation flask along with 75 mL of water. The mixture was then distilled using a 15 cm jacketed Vigreuex column. The fraction boiling between 85-92°C was collected to afford 174.3 g of a water white liquid. The main fraction distilled as an azeotrope with water. This material was found to contain ∼ 28% water. Of the remaining organic material, GLC analysis indicated 90% 3-amino-3-methyl-1-pentyne with most of the remainder being 3-methyl-1-pentyn-3-ol and butanone. The yield of 3-amino-3-methyl-1-pentyne was 60% based on the 3-chloro-3-methyl-1-pentyne starting material.
(1) Triton is a registered trademark of the Union Carbide Chemical & Plastics Co.

### Comparative Example: Formation of 3-Amino-3-methyl-1-pentyne

The procedure used was substantially similar to that employed in Example 2 except that no surfactant was added to the reactor system. The yield of 3-amino-3-methyl-1-pentyne in this case was 39% based on the 3-chloro-3-methyl-1-pentyne starting material.

## Claims

1. A process for the synthesis of an aminoalkyne which comprises the steps of
a. reacting an alkynyl alcohol with HCl to form a chloroalkyne,
b. reacting said chloroalkyne with a water soluble amination agent in the presence of a surfactant to form an aminoalkyne and, optionally,
c. purifying said aminoalkyne by distillation.

2. The process of claim 1 which comprises the steps of
a. reacting an alkynyl alcohol of the formula 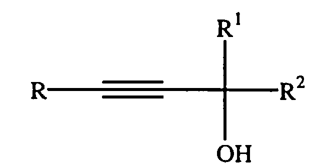 with an aqueous, saturated HCl solution to form a chloroalkyne of the formula 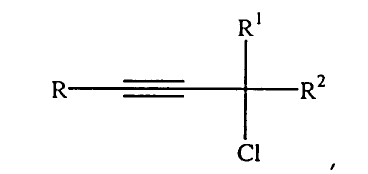
b. reacting said chloroalkyne with a water soluble amination agent of the formula NR³R⁴ in the presence of a non-ionic, cationic or amphoteric surfactant to form an aminoalkyne of the formula 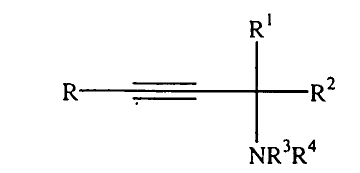 and, optionally,
c. purifying said aminoalkyne by distillation;
wherein
R is a hydrogen atom, alkyl, cycloalkyl, cycloalkylalkyl or aralkyl;
R¹ and R² are each independently alkyl, cycloalkyl, cycloalkylalkyl, aralkyl or, together with the carbon atom to which they are attached, form cycloalkyl; and
R³ and R⁴ are each independently a hydrogen atom or a lower alkyl,
wherein lower alkyl is straight or branched chain (C₁-C₄)alkyl.

3. The process of claim 2 wherein R is a hydrogen atom or lower alkyl.

4. The process of claim 2 wherein R¹ and R² are independently lower alkyl or, together with the carbon atom to which they are attached, form cyclopentyl or cyclohexyl.

5. The process of claim 2 wherein R³ and R⁴ are independently a hydrogen atom or lower alkyl.

6. The process of claim 2 wherein the surfactant is non-ionic.

7. The process of claim 3 wherein R is a hydrogen atom.

8. The process of claim 4 wherein R¹ and R² are independently methyl or ethyl.

9. The process of claim 5 wherein R³ and R⁴ are both hydrogen atoms.

10. The process of claim 8 wherein R¹ is methyl and R² is methyl or ethyl.

11. A process for the synthesis of an aminoalkyne from a chloroalkyne comprising reacting said chloroalkyne with a water soluble amination agent in the presence of a surfactant.

12. The process of claim 11 wherein the chloroalkyne having the formula 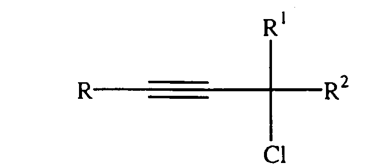 is reacted with a water soluble amination agent of the formula NR³R⁴ in the presence of a non-ionic, cationic or amphoteric surfactant to form an aminoalkyne of the formula 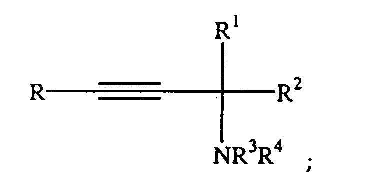 wherein
R is a hydrogen atom, alkyl, cycloalkyl, cycloalkylalkyl or aralkyl;
R¹ and R² are each independently alkyl, cycloalkyl, cycloalkylalkyl, aralkyl or, together with the carbon atom to which they are attached, form cycloalkyl; and
R³ and R⁴ are each independently a hydrogen atom or a lower alkyl,
wherein
lower alkyl is straight or branched chain (C₁-C₄)alkyl.

13. The process of claim 12 wherein R is a hydrogen atom or lower alkyl.

14. The process of claim 12 wherein R¹ and R² are independently lower alkyl or, together with the carbon atom to which they are attached, form cyclopentyl or cyclohexyl.

15. The process of claim 12 wherein R³ and R⁴ are independently a hydrogen atom or lower alkyl.

16. The process of claim 12 wherein the surfactant is non-ionic.

17. The process of claim 13 wherein R is a hydrogen atom.

18. The process of claim 14 wherein R¹ and R² are independently methyl or ethyl.

19. The process of claim 15 wherein R³ and R⁴ are both hydrogen atoms.

20. The process of claim 18 wherein R¹ is methyl and R² is methyl or ethyl.

21. The process of claim 6 wherein the surfactant is an alkylphenoxy polyethoxy ethanol.

22. The process of claim 16 wherein the surfactant is an alkylphenoxy polyethoxy ethanol.

## Patentansprüche

1. Ein Verfahren zur Synthese eines Aminoalkins, welches die Schritte umfaßt
a. Reagieren eines Alkinylalkohols mit HCl unter Bildung eines Chloralkins,
b. Reagieren des Chloralkins mit einem wasserlöslichen Aminierungsmittel in Anwesenheit eines oberflächenaktiven Stoffes unter Bildung eines Aminoalkins, und optional,
c. Reinigen des Aminoalkins durch Destillation.

2. Das Verfahren nach Anspruch 1, welches die Schritte umfaßt
a. Reagieren eines Alkinylalkohols der Formel 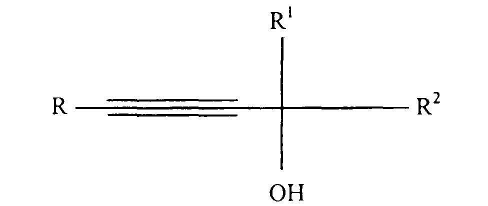 mit einer wässrigen, gesättigten HCl-Lösung unter Bildung eines Chloralkins der Formel 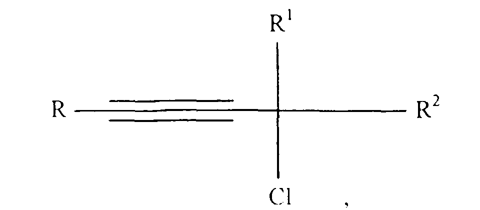
b. Reagieren des Chloralkins mit einem wasserlöslichen Aminierungsmittel der Formel NR³R⁴ in Anwesenheit eines nichtionischen, kationischen oder amphoteren oberflächenaktiven Mittels unter Bildung eines Aminoalkins der Formel 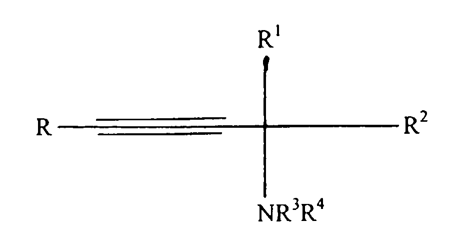 und, optional,
c. Reinigen des Aminoalkins durch Destillation;
worin
R ein Wasserstoffatom, Alkyl, Cycloalkyl, Cycloalkylalkyl oder Aralkyl ist,
R¹ und R² unabhängig voneinander ein Alkyl, Cycloalkyl, Cycloalkylalkyl, Aralkyl sind oder zusammen mit dem Kohlenstoffatom, an das sie gebunden sind, ein Cycloalkyl bilden; und
R³ und R⁴ unabhängig voneinander ein Wasserstoffatom oder Niederalkyl sind,
worin das Niederalkyl ein gerad- oder verzweigtkettiges (C₁ - C₄)Alkyl ist.

3. Das Verfahren nach Anspruch 2, worin R ein Wasserstoffatom oder Niederalkyl ist.

4. Das Verfahren nach Anspruch 2, worin R¹ und R² unabhängig voneinander ein Niederalkyl sind oder zusammen mit dem Kohlenstoffatom, an das sie gebunden sind, ein Cyclopentyl oder Cyclohexyl bilden.

5. Das Verfahren nach Anspruch 2, worin R³ und R⁴ unabhängig voneinander ein Wasserstoffatom oder Niederalkyl sind.

6. Das Verfahren nach Anspruch 2, worin das oberflächenaktive Mittel nichtionisch ist.

7. Das Verfahren nach Anspruch 3, worin R ein Wasserstoffatom ist.

8. Das Verfahren nach Anspruch 4, worin R¹ und R² unabhängig voneinander Methyl oder Ethyl sind.

9. Das Verfahren nach Anspruch 5, worin sowohl R³ als auch R⁴ Wasserstoffatome sind.

10. Das Verfahren nach Anspruch 8, worin R¹ Methyl ist und R² Methyl oder Ethyl ist.

11. Ein Verfahren zur Synthese eines Aminoalkins aus einem Chloralkin, umfassend das Reagieren des Chloralkins mit einem wasserlöslichen Aminierungsmittel in Anwesenheit eines oberflächenaktiven Mittels.

12. Das Verfahren nach Anspruch 11, worin das Chloralkin mit der Formel 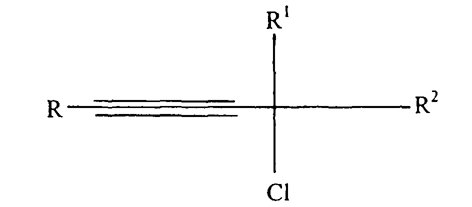 mit einem wasserlöslichen Aminierungsmittel der Formel NR³R⁴ in Anwesenheit eines nichtionischen, kationischen oder amphoteren oberflächenaktiven Mittels unter Bildung eines Aminoalkins der Formel 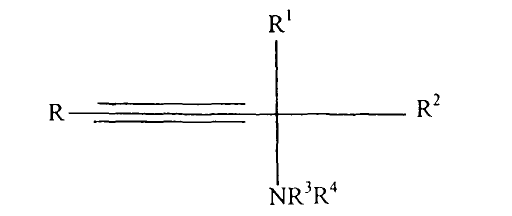 umgesetzt wird;
worin
R ein Wasserstoffatom, Alkyl, Cycloalkyl, Cycloalkylalkyl oder Aralkyl ist;
R¹ und R² unabhängig voneinander ein Alkyl, Cycloalkyl, Cycloalkylalkyl, Aralkyl sind oder zusammen mit dem Kohlenstoffatom, an das sie gebunden sind, ein Cycloalkyl bilden; und
R³ und R⁴ unabhängig voneinander ein Kohlenstoffatom oder Niederalkyl sind, worin das Niederalkyl ein gerad- oder verzweigtkettiges (C₁ - C₄)Alkyl ist.

13. Das Verfahren nach Anspruch 12, worin R ein Wasserstoffatom oder Niederalkyl ist.

14. Das Verfahren nach Anspruch 12, worin R¹ und R² unabhängig voneinander ein Niederalkyl sind oder zusammen mit dem Kohlenstoffatom, an das sie gebunden sind, ein Cyclopentyl oder Cyclohexyl bilden.

15. Das Verfahren nach Anspruch 12, worin R³ und R⁴ unabhängig voneinander ein Wasserstoffatom oder Niederalkyl sind.

16. Das Verfahren nach Anspruch 12, worin das oberflächenaktive Mittel nichtionisch ist.

17. Das Verfahren nach Anspruch 13, worin R ein Wasserstoffatom ist.

18. Das Verfahren nach Anspruch 14, worin R¹ und R² unabhängig voneinander Methyl oder Ethyl sind.

19. Das Verfahren nach Anspruch 15, worin sowohl R³ als auch R⁴ Wasserstoffatome sind.

20. Das Verfahren nach Anspruch 18, worin R¹ Methyl ist und R² Methyl oder Ethyl ist.

21. Das Verfahren nach Anspruch 6, worin das oberflächenaktive Mittel ein Alkylphenoxypolyethoxyethanol ist.

22. Das Verfahren nach Anspruch 16, worin das oberflächenaktive Mittel ein Alkylphenoxypolyethoxyethanol ist.

## Revendications

1. Procédé pour la synthèse d'un aminoalcyne, qui comprend les étapes consistant à
a. faire réagir un alcool alcynylique avec du HCl pour former un chloroalcyne,
b. faire réagir ledit chloroalcyne avec un agent d'amination soluble dans l'eau, en présence d'un tensioactif pour former un aminoalcyne et, éventuellement,
c. purifier ledit aminoalcyne par distillation.

2. Procédé selon la revendication 1, qui comprend les étapes consistant à:
a. faire réagir un alcool alcynylique de formule 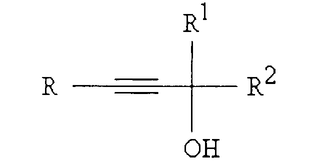 avec une solution aqueuse saturée de HCl pour former un chloroalcyne de formule 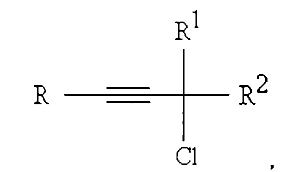
b. faire réagir ledit chloroalcyne avec un agent d'amination soluble dans l'eau de formule NR³R⁴ en présence d'un tensioactif non ionique, cationique ou amphotère pour former un aminoalcyne de formule 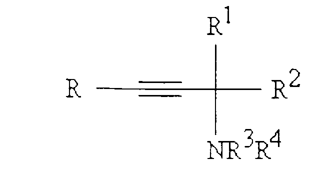 et, éventuellement
c. purifier ledit aminoalcyne par distillation;
dans laquelle
R est un atome d'hydrogène ou un groupe alkyle, cycloalkyle, cycloalkylalkyle ou aralkyle;
R¹ et R² représentent chacun indépendamment un groupe alkyle, cycloalkyle, cycloalkylalkyle, aralkyle ou forment, avec l'atome de carbone auquel ils sont liés, un groupe cycloalkyle; et
R³ et R⁴ représentent chacun indépendamment un atome d'hydrogène ou un groupe alkyle inférieur,
le groupe alkyle inférieur étant un groupe alkyle en C₁-C₄ à chaîne droite ou ramifiée.

3. Procédé selon la revendication 2, dans lequel R est un atome d'hydrogène ou un groupe alkyle inférieur.

4. Procédé selon la revendication 2, dans lequel R¹ et R² sont indépendamment un groupe alkyle inférieur ou forment, avec l'atome de carbone auquel ils sont liés, un groupe cyclopentyle ou cyclohexyle.

5. Procédé selon la revendication 2, dans lequel R³ et R⁴ sont indépendamment un atome d'hydrogène ou un groupe alkyle inférieur.

6. Procédé selon la revendication 2, dans lequel le tensioactif est un tensioactif non ionique.

7. Procédé selon la revendication 3, dans lequel R est un atome d'hydrogène.

8. Procédé selon la revendication 4, dans lequel R¹ et R² sont indépendamment un groupe méthyle ou éthyle.

9. Procédé selon la revendication 5, dans lequel R³ et R⁴ sont tous deux des atomes d'hydrogène.

10. Procédé selon la revendication 8, dans lequel R¹ est un groupe méthyle et R² est un groupe méthyle ou éthyle.

11. Procédé pour la synthèse d'un aminoalcyne à partir d'un chloroalcyne, comprenant l'étape qui consiste à faire réagir ledit chloroalcyne avec un agent d'amination soluble dans l'eau, en présence d'un tensioactif.

12. Procédé selon la revendication 11, dans lequel le chloroalcyne de formule 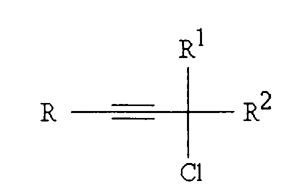 est mis à réagir avec un agent d'amination soluble dans l'eau de formule NR³R⁴ en présence d'un tensioactif non ionique, cationique ou amphotère pour former un aminoalcyne de formule 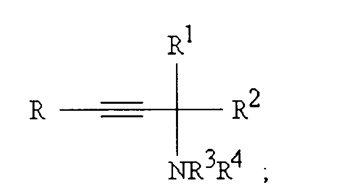 dans laquelle
R est un atome d'hydrogène ou un groupe alkyle, cycloalkyle, cycloalkylalkyle ou aralkyle;
R¹ et R² représentent chacun indépendamment un groupe alkyle, cycloalkyle, cycloalkylalkyle, aralkyle ou forment avec l'atome de carbone auquel ils sont liés un groupe cycloalkyle; et
R³ et R⁴ représentent chacun indépendamment un atome d'hydrogène ou un groupe alkyle inférieur,
le groupe alkyle inférieur étant un groupe alkyle en C₁-C₄ à chaîne droite ou ramifiée.

13. Procédé selon la revendication 12, dans lequel R est un atome d'hydrogène ou un groupe alkyle inférieur.

14. Procédé selon la revendication 12, dans lequel R¹ et R² sont indépendamment un groupe alkyle inférieur ou forment avec l'atome de carbone auquel ils sont liés, un groupe cyclopentyle ou cyclohexyle.

15. Procédé selon la revendication 12, dans lequel R³ et R⁴ sont indépendamment un atome d'hydrogène ou un groupe alkyle inférieur.

16. Procédé selon la revendication 12, dans lequel le tensioactif est un tensioactif non ionique.

17. Procédé selon la revendication 13, dans lequel R est un atome d'hydrogène.

18. Procédé selon la revendication 14, dans lequel R¹ et R² sont indépendamment un groupe méthyle ou éthyle.

19. Procédé selon la revendication 15, dans lequel R³ et R⁴ sont tous deux des atomes d'hydrogène.

20. Procédé selon la revendication 18, dans lequel R¹ est un groupe méthyle et R² est un groupe méthyle ou éthyle.

21. Procédé selon la revendication 6, dans lequel le tensioactif est un alkylphénoxypolyéthoxyéthanol.

22. Procédé selon la revendication 16, dans lequel le tensioactif est un alkylphénoxypolyéthoxyéthanol.
